# EUROPEAN PATENT SPECIFICATION

(11) **EP 2 940 122 B1**
(45) Date of publication and mention of the grant of the patent: **22.05.2019**
(21) Application number: 15162520.9
(22) Date of filing: 06.04.2015
(51) Int. Cl.: C12N 1/14, C12N 1/20, C22B 3/18, C12R 1/44, C12R 1/46, C12R 1/645

(54) **CACO3 DISSOLVING BACTERIAL STRAINS AND FUNGUS**
CACO3 AUFLÖSENDE BAKTERIENSTÄMME UND PILZ
SOUCHES BACTÉRIENNES ET CHAMPIGNON DISSOLVANT LE CACO3

(30) Priority: 28.04.2014 TR 201404763
(43) Date of publication of application: 04.11.2015
(73) Proprietor: Yeditepe Universitesi, 34755 Istanbul (TR)
(72) Inventor: Sahin, Fikrettin, 34755 Istanbul (TR); Yilmazer, Gonca Altin, 34755 Istanbul (TR); Yazici, Müge, 34755 Istanbul (TR); Eroglu, Seckin, 35590 Izmir (TR)
(74) Representative: Dericioglu Kurt, Ekin

(56) References cited:
- Gulluce M. et al.: "Conventional and Molecular Identification of bacteria with magnesite enrichment potential from local quarries in Erzurum", Geomicrobiology Journal, vol. 31 4 March 2014 (2014-03-04), pages 445-451, XP055204812, DOI: 10.1080/01490451.2013.791357 Retrieved from the Internet: URL:http://www.tandfonline.com/loi/ugmb20 [retrieved on 2015-07-28]
- ORHAN FURKAN ET AL: "Identification and application of CaCO3 dissolving bacteria in magnesite quarries", CURRENT OPINION IN BIOTECHNOLOGY, vol. 24, no. Suppl. 1, July 2013 (2013-07) , page S31, XP002742743, & EUROPEAN BIOTECHNOLOGY CONGRESS; BRATISLAVA, SLOVAKIA; MAY 16 -18, 2013
- YANMIS DERYA ET AL: "The enrichment potential of magnesite by using some Actinomycetes isolated from caves", CURRENT OPINION IN BIOTECHNOLOGY, vol. 22, no. Suppl. 1, September 2011 (2011-09), page S62, XP002742744, & EUROPEAN BIOTECHNOLOGY CONGRESS; ISTANBUL, TURKEY; SEPTEMBER 28 -OCTOBER 01, 2011
- GANGAVARAPU SUBRAHMANYAM ET AL: "Carbonate-Dissolving Bacteria from "Milolite", a Bioclastic Limestone, from Gopnath, Gujarat, Western India", MICROBES AND ENVIRONMENTS, vol. 27, no. 3, 1 January 2012 (2012-01-01), pages 334-337, XP55204815, ISSN: 1342-6311, DOI: 10.1264/jsme2.ME11347

## Description

### Field of the Invention

The present invention relates to bacterial strains and fungal isolates which can dissolve the CaCO₃ mineral in the magnesite ore via biological mechanisms.

### Background of the Invention

Magnesite is a mineral, which theoretically comprises 47.8% MgO and 52.2% CO₂ and a trace amount of Fe₂O₃, and whose specific gravity varies in the range of 2.9-3.1 g/cm³ depending on its content. Magnesite, which is the main source of magnesium element and various compounds thereof can be used in many fields from production of refractory brick to pharmaceutical products (Ozdemir et al., 2009). Due to the fact that magnesium is a reactive element, magnesite ore is not present in any source at a purity that allows it to be used directly (Bentl 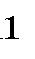 et al., 2004). Magnesite ores contain various amounts of Ca, Si, Fe, Mg and Al elements as impurities (Gürcan et al., 2012). The impurities around the magnesite should be removed in order for it to become commercially usable. As the purity of the mineral increases, its value increases as well. Therefore the mineral is subjected to various physical and chemical processes such as magnetic separation, hand sorting, heavy medium separation and flotation (Gence, 2001). Magnesite processing technology starts with extracting ore from the mineral deposit. The mineral is subjected to various enrichment processes after being extracted from the deposit. Enrichment process is separation of the ore from the gang minerals. Characteristic gang minerals of crystalline magnesite are dolomite, biotite, garnet, talc, quartz; and of gel magnesite are serpentine and opal. Generally triage (hand sorting), heavy medium enrichment, magnetic separation, electrostatic separation, flotation enrichment, calcination and optical enrichment methods are used for enrichment of magnesite (Kocaefe 1982; Erdo 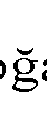 an and Y 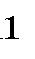 ld 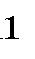 z 1995). The method of heavy medium enrichment is based on the density difference between the magnesite material and gang minerals. Ferrosilicon (6,8 gr/cm³), which is a very dense material, is used for this process. With this method, substances that are denser than ferrosilicon precipitate while the lighter substances float. Hand sorting is an observational method, which is based on man power. Magnesite is separated from the other minerals according to color differences.

Magnetic enrichment method is a type of separation based on magnetic properties of the minerals. Magnetic enrichment is used for separating ferromagnetic and paramagnetic gang materials from magnesite since magnesite is a non-metal and non-magnetic mineral. Flotation is an enrichment method for separating magnesite from calcium carbonate and silica wherein oleic acid and a small amount of carbon dioxide are used as media. The most important disadvantage of this method is cost. Due to the high cost of this method, magnetic enrichment and hand sorting methods are preferred more (Özdemir et al., 2009). Calcination is the process of obtaining a more dense and solid magnesite by heating to high temperatures and removing carbondioxide. When magnesite is heated to 650-1100°C in a controlled manner, calcined magnesite is obtained. This magnesite is reactive. When magnesite is heated up to 1400-1800°C, sintered magnesite is obtained. This magnesite is slightly reactive (Chen and Tao, 2004). Due to economic reasons, the most common ones of these enrichment methods are hand sorting, magnetic separation and calcination (Gence, 2001; Demir and Dönmez, 2009). Although these physical and chemicals methods are widely used for treatment of sediments contaminated with metals, they have disadvantages such as the high cost and high amount of waste resulting due to the facts that low quality ores cannot be enriched, the amount of recovered product is low, refractory materials is used in many processes and high amounts of energy are required (Rulkens 1995; Sever 2006). In addition, studies are conducted by the researchers in many countries on biological applications in mining for the last 40 years (Hoque and Philip, 2011). This process, which is defined as biomining, has provided an alternative to the physical and chemical methods (Rawlings and Johnson, 2007; Brierley, 2008; Appana et al., 2011). Biomining, which is an economic method even in environment friendly and low quality mines as opposed to the conventional methods, is still being used in copper, gold, silver, cobalt, nickel, molybdenum, zinc and lead mines with *cidithiobacillus ferrooxidans, Acidithiobacillus thiooxidans, Leptospirillum ferrooxidans* ve *Leptospirillum ferriphilum* microorganisms (Baret and Hughes, 1993; Watling, 2006; Siddiqui et al., 2009;Hoque and Philip, 2011).

The Chinese patent document no. CN102976640, an application in the state of the art, discloses about obtaining high-quality magnesite and calcination carried out at super high-temperature in order to obtain it.

The Chinese Patent document no CN103241751, an application known in the state of the art, discloses about obtaining a high-purity magnesium oxide. The said document also discloses about discharge of a high amount of carbon dioxide and removal thereof from the environment.

The Chinese patent document no. CN102126734, an application in the state of the art, discloses that washing and neutralization processes are carried out for obtaining magnesite.

Gulluce M. et al; discloses conventional and molecular identification of bacteria with magnesite enrichment potential from local quarries in Erzurum.

Orhan Furkan et. al; discloses identification and application of CaCO3 dissolving bacteria in magnesite quarries.

Yanmis Derya et. al; the enrichment potential of magnesite by using some Actinomycetes isolated from caves.

Gangavarapu subrahmanyam et. al; carbonate-dissolving bacteria from "milolite", a bioclastic Limestone from India.

### Summary of the Invention

The objective of the present invention is to provide new bacterial strains and fungal isolates which enable to enrich magnesite.

Another objective of the present invention is to provide new bacterial strains and fungal isolates which provide more economic and environment friendly dissolving process than the known methods.

A further objective of the present invention is to provide new bacterial strains and fungal isolates which enable to enrich low quality mines.

Another objective of the present invention is to provide new bacterial strains and fungal isolates wherein chemicals are not used during dissolving process, and which do not discharge environmentally hazardous wastes.

Another objective of the present invention is to provide new bacterial strains and fungal isolates which do not consume high energy during decalcification.

A further objective of the present invention is to provide new bacterial strains and fungal isolates which eliminates the cost for transporting the mine as it can be applied in a mineral deposit.

Another objective of the present invention is to provide new bacterial strains and fungal isolates which decrease labor and reduce worker cost since it eliminates a great deal of process steps in comparison to the known methods in decalcification.

### Detailed Description of the Invention

'Bacterial strains and fungal isolates dissolving CaCO3 biologically" developed to fulfill the objective of the present invention are illustrated in the accompanying figures wherein,
**Figure 1** is the graphical representation of magnesite demineralization of *Enterecoccus* spp. strain and the free Mg and Ca content.
**Figure 2** is the graphical representation of magnesite demineralization of *Lactococcus* spp. strain and the free Mg and Ca content.
**Figure 3** is the graphical representation of magnesite demineralization of *Staphylococcus* spp. strain and the free Mg and Ca content.
**Figure 4** is the graphical representation of magnesite demineralization of *Neurospora* spp. strain and the free Mg and Ca content.

Three microbial bacteria strains are used for decalcifying the magnesite according to the present invention. One of the strains of the present invention was deposited in United States Department of Agriculture Research, Education and Economics Agricultural Research Service on November 29, 2008 Accordingly, deposit number of the strain named *Lactococcus garviae* A1 was recorded as NRRL B-50196. The other two of the strains of the present invention were deposited in Leibniz Institute DSMZ German Collection of Microorganisms and Cell Cultures (Leibniz-Institut DSMZ - Deutsche Sammlung von Mikroorganismen und Zellkulturen GmbH (DSMZ)) on October 17, 2013. Accordingly, deposit numbers of *Staphylococcus warneri* GSK1 strain and *Neurospora tetrasperma* GNT1 isolate were recorded as DSM 27873 and DSM 27874 respectively.

In order to obtain high purity magnesium from magnesite, CaCO₃ (calcium carbonate) in the medium should be removed.

*Enterococcus* spp.and *Lactococcus* spp. strains were isolated from the rock. *Staphylococcus warneri* and *Neurospora tetrasperma* were isolated from a magnesite mine. While the isolates *Enterococcus spp. Lactococcus* spp., *Staphylococcus warneir* are gram-positive bacteria, *Neurospora tetrasperma* is a fungus. The microorganisms of the present invention are mesophilic as they are isolated from natural environment.

### Experimental studies

Granulated magnesite (MgCO₃) mineral was used in the experimental studies. By carrying out experimental studies in a medium containing calcium carbonate, a medium with magnesite was prepared. In preparation of the medium; 5g yeast extract, 5g glucose, 6g magnesite (MgCO₃) and 500 ml water were used. The medium was sterilized using an autoclave. *Enterococcus* spp. *Lactococcus* spp. *Staphylococcus warneri* strains and *Neurospora tetrasperma* isolate were inoculated to the magnesite-containing medium and was incubated at 30°C and 150 rpm (Sharafi et al. 2010). Samples were taken during the study at certain intervals in order to make measurements. The elemental analyses were performed with ICP-MS device. The samples, which were taken in a sterile cabin, were filtered through a 0.2 µm filter. The filtered samples were diluted to 100µl/10ml (filtrate/ 2% HNO₃) and Mg and Ca amount were measured by the ICP-MS device.

While these microorganisms are used alone in purification of magnesium, they can also be used in different combinations with each other.

### Experiment results

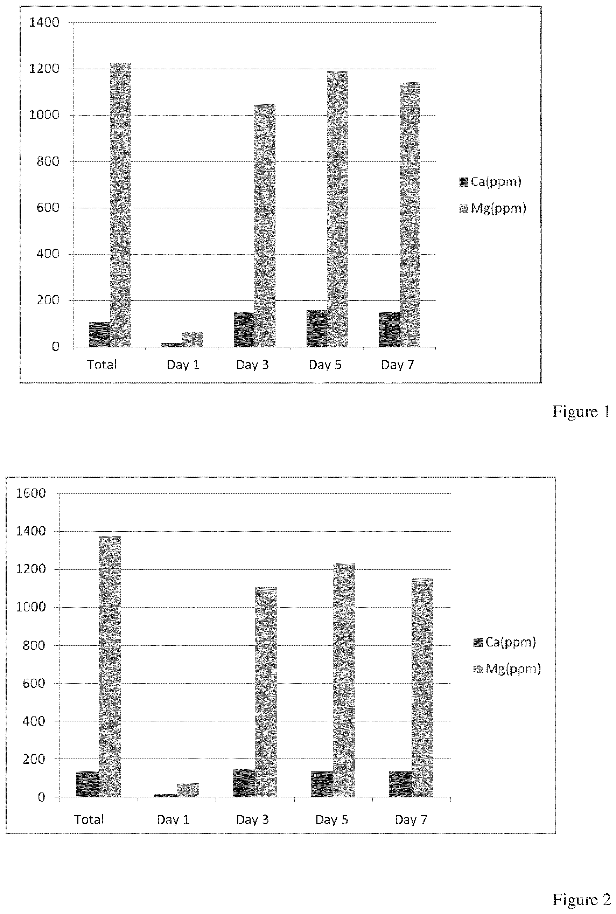

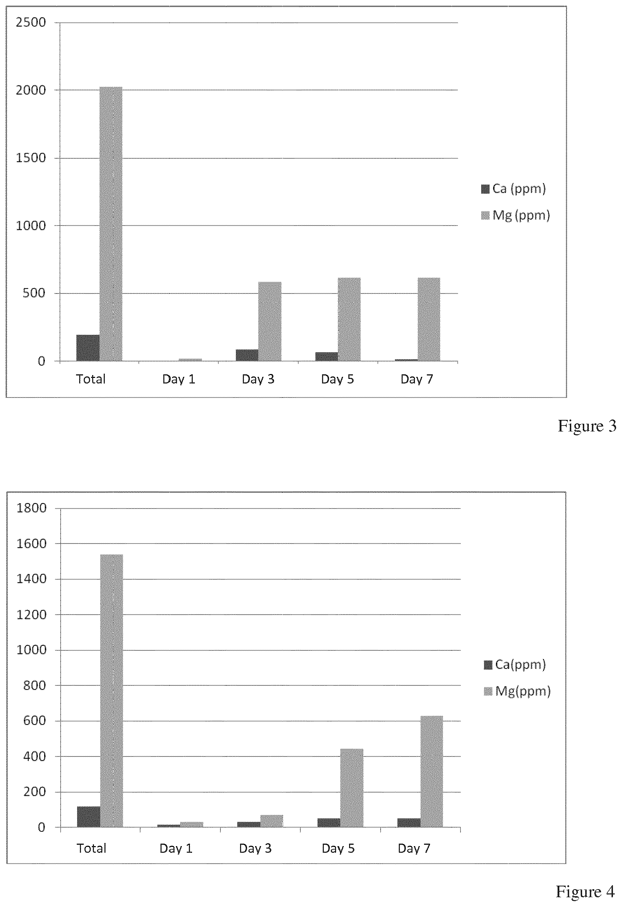

*Enterococcus* spp., *Lactococcus* spp., *Staphylococcus warneir* strains and *Neurospora tetrasperma* isolate were incubated in the magnesite-containing medium; and elemental analyses were conducted by means of ICP-MS for measuring free Mg and Ca amount (Danadurai et al., 2011). The results for the magnesite demineralization at the end of the measurement are shown in Figure 1, 2, 3, 4.

Free magnesium that is liberated as a result demineralization is shown in Figure 1. The free calcium content increasing at the same time shows that *Enterecoccus* spp. strain dissolves CaCO₃. While the columns that we name as the total show the initial Mg and Ca contents, the other columns show the free Mg and Ca content which are liberated by being dissolved by microorganisms in time. The same results were observed when the other microorganisms of the present invention were used (Figure 2, 3, 4).

The microorganisms of the present invention, can be in the form of a liquid or solid solution, powder, granule.

Bacteria microorganisms are applied for mine demineralization, elimination of the clogging in irrigation systems, and decalcifying alkaline soils making them fertile.

### References

Appanna, V. , Lemire, J.,Hnatiuk, R., 2011. "Biomining: A Gren Technology to Mine Valuable Metals". Available on site
Barrett, J.,Hughes, M.N., Karavaiko, G.I.,Spencer, P.A., (Eds.), "Metal Extraction by Bacterial Oxidation of Minerals" Ellis Horwood Limited, 1993.
Bentl 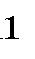 , I., Erdg 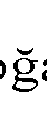 an, N., Birici, B., Topal, U., and 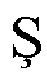 ahbaz, O., 2004. "Manyezit ara ürünün kalsinasyon-manyetik ay 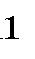 rma yöntemleriyle zenginle 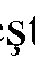 tirilmesi" J. *Endüstriyel Hammaddeler Sempozyumu, İzmir, Türkiye*
Brierley, C. L., 2008. "How will biomining be applied in future?". Trans. Nonferrous.Met. Soc. Of China, 1302-1310
Chen, G.,and Tao, D., 2004. "Effect of solution chemistry on flotability of magnesite and dolomite". Int. J. Miner. Process. 74:343- 357
Danadurai, K.S.K.,Chellam, S., Lee, C., and Fraser, M.P., 2011."Trace elemental analysis of airborne particulate matter using dynamic reaction cell inductively coupled plasma - masss pectrometry: Application to monitoring episodic industrial emission events". AnalyticaChimicaActa686 : 40-49
Erdo 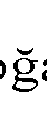 an N, Y 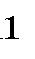 ld 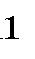 z R, 1995. *Manyezit ve Bazit Refrakter Malzeme Teknolojisi,* Kuma 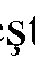 , Kütahya, 150s.
Gence, N., 2001. "Enrichment of magnesiteore". Eng.&Arch.Fac.OsmangaziUniversity, Vol.XIV(2*)*
Gürcan, H., Sesver, A., Ozdemir, N., Ozdemir, B., 2012."Cryptocrystalline and Macrocrystalline Magnesite Ores: Comparing Microstructures and Thermal Behaviour".http://www.arber.com.tr/imps2012.org/proceedingsebook/ Abstract/ab sfilAbstractSubmissionFullContent386.pdf
Hoque, E.,and Philip, O.J., 2011. "Biotechnological recovery of heavy metals from secondary sources-An overview". MaterialsScienceandEngineering C, 57-66
Kocaefe M. 1982. Manyezit envanteri, MTA Yay 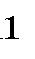 nlar 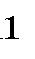 , Ankara.
Ozdemir, M., Çakιr, D., K 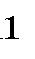 pçak, İ., 2009."Magnesium recovery from magnesite tailings by acid leaching and production of magnesium chloride hexahydrate from leaching solution by evaporation". *Int. J. Miner.Process.* 93:209-212
Rawlings, D. E., and Johnson, D. B., 2007."The microbiology of biomining: development and optimization of mineral-oxidizing microbial consortia". Microbiology, 153: 315-324
Rulkens WH, Grotenhuis, JTC, Tichy R, 1995. Methods of cleaning contaminated soils and sediments. In: Salomons W. Förstner U, Mader P. (Eds.) HeavyMetals. Springer, Berlin, pp, 151-191.
Sever E, 2006. İskenderun Demir Çelik Fabrikasι At 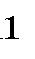 klarιna Biyoliç Uygulamasι. Yükseklisans Tezi, Çukurova Üniversitesi, Fen Bilimleri Enstitüsü, Adana, Türkiye.
Sharafi, S.M.,Rasooli, I., Beheshti-Maal, K., 2010. "Isolation, characterization and optimization of indigenous acetic acid bacteria and evaluation of their preservation methods". Iranian Journal of Microbiology,2 : 41-48
Siddiqui, M. H., Kumar, A., Kesari, K. K., and Arif, J. M., 2009."Biomining - A UsefulApproachToward Metal Extraction". American-EurasianJournal of Agronomy 2, 2:84-88
Watling, H R., 2006."The bioleaching of sulphide minerals with emphasis on copper sulphides-A review". Hydrometallurgy, 84:81-108

## Claims

1. Calcium carbonate and magnesite dissolving A1 bacterial strain which is deposited with the number NRRL B-50196.

2. Calcium carbonate and magnesite dissolving GSK1 bacterial strain which is deposited with the number DSM 27873.

3. Calcium carbonate and magnesite dissolving GNT1 fungal isolate which is deposited with the number DSM 27874.

4. Calcium carbonate dissolving bacterial strains and fungal isolate according to any one of Claims 1 to 3, which can be applied together in combination.

5. Calcium carbonate dissolving bacterial strains and fungal isolate according to any one of Claims 1 to 4, which can be applied in fields of mine demineralization, elimination of clogging in irrigation systems, and decalcifying alkaline soils thereby making them fertile.

## Patentansprüche

1. Kalziumkarbonat und Magnesit lösender A1-Bakterienstamm, der unter der Nummer NRRL B-50196 abgeschieden ist.

2. Kalziumkarbonat und Magnesit lösender GSK1-Bakterienstamm, der unter der Nummer DSM 27873 abgeschieden ist.

3. Kalziumkarbonat und Magnesit lösendes GNT1-Pilzisolat, das unter der Nummer DSM 27874 abgeschieden ist.

4. Kalziumkarbonat lösende Bakterienstämme und Pilzisolat nach einem der Ansprüche 1 bis 3, die zusammen in Kombination angewendet werden können.

5. Kalziumkarbonat lösende Bakterienstämme und Pilzisolate nach einem der Ansprüche 1 bis 4, die in Bereichen der Grubenentsalzung, der Beseitigung von Verstopfungen in Bewässerungssystemen und der Entkalkung alkalischer Böden angewendet werden können, indem sie diese fruchtbar machen.

## Revendications

1. La souche bactérienne A1 dissolvant carbonate de calcium et de magnésite qui est déposée sous le numéro NRRL B-50196.

2. La souche bactérienne GSK1 dissolvant carbonate de calcium et de magnésite qui est déposée sous le numéro DSM 27873.

3. Isolat fongique GNT1 dissolvant carbonate de calcium et de magnésite est déposé sous le numéro DSM 27874.

4. Souches bactériennes et isolats fongiques dissolvant carbonate de calcium et de magnésite selon l'une quelconque des revendications 1 à 3, qui peuvent être appliqués ensemble en combinaison.

5. Souches bactériennes et isolats fongiques dissolvant carbonate de calcium et de magnésite selon l'une quelconque des revendications 1 à 4, qui peuvent être appliqués dans les domaines de la déminéralisation des mines, de l'élimination du colmatage des systèmes d'irrigation et de la décalcification des sols alcalins les rendant ainsi fertiles.
